# EUROPÄISCHE PATENTSCHRIFT

(11) **EP 1 585 490 B1**
(45) Veröffentlichungstag und Bekanntmachung des Hinweises auf die Patenterteilung: **26.10.2005**
(21) Anmeldenummer: 03808244.2
(22) Anmeldetag: 18.12.2003
(51) Int. Cl.: A61K 7/16

(54) **EINFÄRBEMITTEL IN TABLETTENFORM ZUR SICHTBARMACHUNG VON PLAQUE-BILDUNG AUF ZÄHNEN SOWIE VERFAHREN ZUR HERSTELLUNG EINES SOLCHEN EINFÄRBEMITTELS**
STAINING AGENT IN TABLET FORM FOR MAKING VISIBLE THE FORMATION OF DENTAL PLAQUE AND METHOD FOR PRODUCING SUCH A TINTING AGENT
AGENT DE COLORATION SOUS FORME DE COMPRIMES SERVANT A RENDRE VISIBLE LA FORMATION DE LA PLAQUE DENTAIRE ET PROCEDE DE PRODUCTION D'UN TEL AGENT DE COLORATION

(30) Priorität: 18.12.2002 DE 10259640
(43) Veröffentlichungstag der Anmeldung: 19.10.2005
(73) Patentinhaber: Hager & Werken Gmbh & Co. KG, 47269 Duisbrug (DE)
(72) Erfinder: DEPPE, Marc, 47661 Issum (DE)
(74) Vertreter: Sroka, Peter-Christian
(86) Internationale Anmeldenummer: PCT/DE2003/004187
(87) Internationale Veröffentlichungsnummer: WO 2004/054528

(56) Entgegenhaltungen:
- US-A- 3 723 613
- US-A- 3 903 252
- US-A- 4 347 233
- US-A- 4 431 628
- US-A- 4 459 277
- DATABASE WPI Derwent Publications Ltd., London, GB; AN 1996-318866 XP002280799 "Dental plaque-dyeing compsn. having good dyeing selectivity avoiding dyeing of lips and gums" & JP 08 143477 A (LION CORP), 4. Juni 1996 (1996-06-04)
- DATABASE WPI Derwent Publications Ltd., London, GB; AN 1996-184718 XP002280800 "Plaque staining and disclosing agent" & JP 08 059513 A (LION CORP), 5. März 1996 (1996-03-05)

## Beschreibung

Einfärbemittel zur Sichtbarmachung von Plaque-Bildung auf Zähnen sind als Lösungen, Tabletten, Zahnpasten und in Gelform bekannt.

So ist in der US 3,723,613 A ein in Tablettenform vorliegendes Einfärbemittel bekannt, welches FDC Blue No. 1 und FDC Red No. 3 enthält, um ältere Plaque dunkelblau und neuere Plaque lila-rot anzuzeigen, was wertvolle Hinweise für das Fortschreiten der Plaque-Bildung und für die Behandlung derselben liefert.

In der EP 0 421 838 B1 sind eine Plaque-Bildung anzeigende Mittel als bekannt beschrieben, die als üblichen und wasserlöslichen Farbstoff FDC Red No. 3 enthalten. Als nachteilig wird beschrieben, daß FDC Red No. 3 einen unangenehmen Geschmack hat und in den Vereinigten Staaten in den Jahren 1989 als Bestandteil für kosmetische Produkte verboten worden ist, da seine Gesundheitsunschädlichkeit in Frage gestellt ist. Gemäß EP 0 421 838 B1 wird daher als Austausch-Farbstoff der weniger bitter schmeckende Farbstoff FDC Red No. 40 vorgeschlagen, der bei der Plaque-Einfärbung eine stärkere Farbintensität als andere Plaque-anzeigende Mittel, wie etwa FDC Blue No. 1, FDC Blue No. 2 hat, so daß diese Farbstoffe als Plaque-anzeigende Mittel weniger geeignet sind als FDC Red No. 40.

In der JP 10175835 A ist ein Einfärbemittel zur Sichtbarmachung von Plaque-Bildung auf Zähnen in Form einer Zahnpasta beschrieben, die Phloxin als Farbstoff enthält.

In der US 4,347,233 A ist Phloxin B in Lösung als Karies-Detektor beschrieben.

Die US 3,903,252 A behandelt zur Sichtbarmachung von Plaque-Bildung auf Zähnen ein in Gelform vorliegendes Mittel, das eine pharmakologisch harmlose organische Farbstoffkomponente enthält, die ausgewählt ist, aus der Klasse von FDC Red No. 3, FDC Blue No. 1, FDC Violet No. 1 und andere.

Der Erfindung liegt die Aufgabe zugrunde, ein in Tablettenform vorliegendes und damit für Reihen- bzw. Massenuntersuchungen, zum Beispiel an Schulen, besonders geeignetes Einfärbemittel zu schaffen, welches in gesundheitlicher Hinsicht unschädlich und in der Lage ist, ältere Plaque und neuere Plaque anzuzeigen.

Zur Lösung dieser Aufgabe wird ein in Tablettenform vorliegendes Einfärbemittel zur Sichtbarmachung von Plaque-Bildung auf Zähnen vorgeschlagen, welches Lactose im Bereich von etwa 85 - 95 Gew%, Phloxin B im Bereich von etwa 1,5 - 2,6 Gew-%, FDA Blue No. 1 im Bereich von etwa 2,5 - 3,5 Gew-% sowie Magnesiumstearat im Bereich von 0,15 - 0,25 Gew-% enthält.

Durch die Verwendung von Phloxin B neben FDA Blue No. 1 wird erreicht, daß ältere Plaque dunkelblau und neuere Plaque lila-rot angezeigt wird.

Abgesehen von der medizinischen Indikation für den Zahnarzt läßt sich das erfindungsgemäße, in Tablettenform vorliegende Einfärbemittel unabhängig von einer zahnmedizinischen Untersuchung auch von der Einzelperson als Indikator für eine Plaque-Bildung verwenden, wodurch es praktisch auch eine kosmetische Funktion erfüllt.

Bevorzugte Konzentrationen der einzelnen Stoffe sind in den Unteransprüchen 2 bis 5 behandelt. Das verwendete Phloxin B hat die empirische Formel C₂₀H₂Br₄Cl₄Na₂O₅ mit folgender Strukturformel

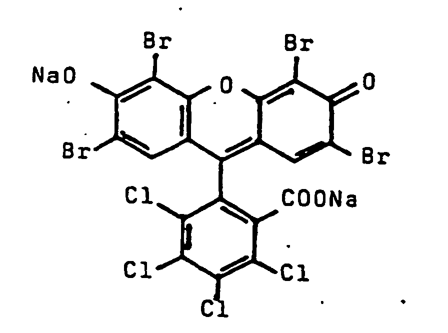

Das verwendete FDA-Blue No. 1 hat die empirische Formel C₃₇H₃₄N₂Na₂O₉S₃ mit der folgenden Strukturformel

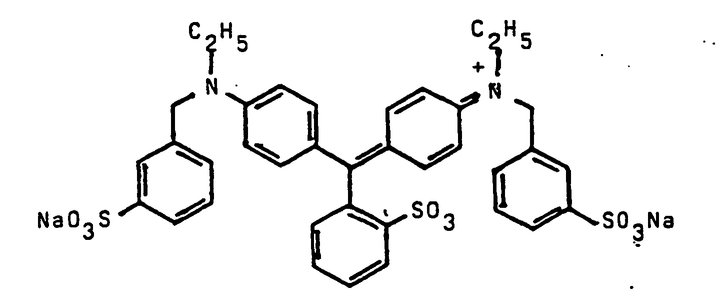

Zur Herstellung der Tabletten wird die Methode der Direkttablettierung gewählt. Dabei werden mikronisierte Farbpartikel der Farbstoffe Phloxin B und FDA-Blue No. 1 mit den Tablettenhilfsmitteln vermischt, wobei darauf zu achten ist, daß die Struktur der Lactose nicht zerstört wird. Die Tabletten werden nach der Tablettierung einer wasserdampfgesättigten Atmosphäre ausgesetzt, vorzugsweise 2 bis 3 Minuten lang, und nehmen dann eine Farbsprenkelung an. Diese Farbsprenkelung tritt ansonsten bei weißen Tabletten während der Lagerung in einer etwas feuchten Atmosphäre auf, was jedoch bei den Testpersonen den Eindruck entstehen läßt, daß die Tabletten bereits gealtert und damit nicht mehr gebrauchsfähig sind.

Das Einfärbemittel enthält weiterhin Pfefferminz- oder Eukalyptus-Aromastoffe im Bereich von etwa 0,3 bis 0,75 Gew.-%.

## Patentansprüche

1. Einfärbemittel in Tablettenform zur Sichtbarmachung von Plaque-Bildung auf Zähnen, enthaltend Lactose im Bereich von etwa 85 bis 95 Gew.-%, Phloxin B im Bereich von etwa 1,5 bis 2,6 Gew.-%, FDA-Blue No. 1 im Bereich von etwa 2,5 bis 3,5 Gew.-% sowie Magnesiumstearat im Bereich von 0,15 bis 0,25 Gew.-%.

2. Einfärbemittel nach Anspruch 1, **dadurch gekennzeichnet, daß** es Pfefferminz- oder Eukalyptus-Aromastoffe im Bereich von etwa 0,3 bis 0,75 Gew.-% enthält.

3. Einfärbemittel nach Anspruch 1 oder 2, **dadurch gekennzeichnet, daß** es Lactose im Bereich von etwa 92 bis 95 Gew.-%, Phloxin B im Bereich von etwa 2 bis 2,2 Gew.-%, FDA-Blue No. 1 im Bereich von etwa 2,7 bis 3,1 Gew.-% sowie Magnesiumstearat im Bereich von 0,18 bis 0,22 Gew.-% enthält.

4. Einfärbemittel nach Anspruch 3, **dadurch gekennzeichnet, daß** es Lactose im Bereich von etwa 94,3 Gew.-%, Phloxin B im Bereich von etwa 2,1 Gew.-%, FDA-Blue No. 1 im Bereich von 2,9 Gew.-%, sowie Magnesiumstearat im Bereich von etwa 0,2 Gew.-% enthält.

5. Einfärbemittel nach einem der Ansprüche 1 bis 4, **dadurch gekennzeichnet, daß** es Pfefferminz- oder Eukalyptus-Aromastoffe im Bereich von 0,5 Gew.-% enthält.

6. Verfahren zur Herstellung eines Einfärbemittels nach einem der Ansprüche 1 bis 5 durch Direkttablettierung, **dadurch gekennzeichnet, daß** man mikronisierte Farbpartikel der Farbstoffe Phloxin B und FDA-Blue No. 1 mit den Tablettenhilfsmitteln Lactose und Magnesiumstearat sowie ggf. Pfefferminz- oder Eukalyptus-Aromastoffe miteinander vermischt und im Anschluß an die Tablettierung die Tabletten ca. 2 bis 3 Minuten einer wasserdampfgesättigten Atmosphäre aussetzt.

7. Verfahren nach Anspruch 6, **dadurch gekennzeichnet, daß** man Lactose im Bereich von etwa 85 bis 95 Gew.-%, Phloxin B im Bereich von etwa 1,5 bis 2,6 Gew.-%, FDA-Blue No. 1 im Bereich von etwa 2,5 bis 3,5 Gew.-% sowie Magnesiumstearat im Bereich von 0,15 bis 0,25 sowie ggf. Pfefferminz- oder Eukalyptus-Aromastoffe im Bereich von etwa 0,3 bis 0,7 Gew.-% miteinander vermischt und diese Mischung tablettiert.

## Claims

1. Staining agent in tablet form for making visible plaque formation on teeth, containing lactose in the range of approximately 85 to 95 % by weight, phloxin B in the range of approximately 1.5 to 2.6 % by weight, FDA Blue No. 1 in the range of approximately 2.5 to 3.5 % by weight, as well as magnesium stearate in the range of 0.15 to 0.25 % by weight.

2. Staining agent according to claim 1, **characterized in that** it contains peppermint or eucalyptus flavoring agents in the range of approximately 0.3 to 0.75 % by weight.

3. Staining agent according to claim 1 or 2, **characterized in that** it contains lactose in the range of approximately 92 to 95 % by weight, phloxin B in the range of approximately 2 to 2.2 % by weight, FDA Blue No. 1 in the range of approximately 2.7 to 3.1 % by weight as well as magnesium stearate in the range of 0.18 to 0.22 % by weight.

4. Staining agent according to claim 3, **characterized in that** it contains lactose in the range of approximately 94.3 % by weight, phloxin B in the range of approximately 2.1 % by weight, FDA Blue No. 1 in the range of 2.9 % by weight, as well as magnesium stearate in the range of approximately 0.2 % by weight.

5. Staining agent according to one of the claims 1 to 4, **characterized in that** it contains peppermint or eucalyptus flavoring agents in the range of 0.5 % by weight.

6. Method for producing a staining agent according to one of the claim 1 to 5 by direct tablet-compressing, **characterized in that** micronized dye particles of the dyes phloxin B and FDA Blue No. 1 are mixed with the tableting assistent agents lactose and magnesium stearate as well as, optionally, peppermint or eucalyptus flavoring agents, and, subsequent to tableting, the tablets are exposed to a water vapor-saturated atmosphere for approximately 2 to 3 minutes.

7. Method according to claim 6, **characterized in that** lactose in the range of approximately 85 to 95 % by weight, phloxin B in the range of approximately 1.5 to 2.6 % by weight, FDC Blue No. 1 in the range of approximately 2.5 to 3.5 % by weight, as well as magnesium stearate in the range of 0.15 to 0.25, as well as, optionally, peppermint or eucalyptus flavoring agents in the range of approximately 0.3 to 0.7 % by weight are mixed together and this mixture is tableted.

## Revendications

1. Agent de coloration sous forme de comprimé, servant à rendre visible la formation de la plaque dentaire, contenant du lactose dans l'intervalle allant d'environ 85 à 95% en poids, la phloxine B dans l'intervalle allant d'environ 1,5 à environ 2,6% en poids, le Bleu FDA N°1 dans l'intervalle allant d'environ 2,5 à environ 3,5% en poids, ainsi que le stéarate de magnésium dans l'intervalle allant d'environ 0,15 à environ 0,25% en poids.

2. Agent de coloration selon la revendication 1, **caractérisé en ce qu'**il contient un arôme de menthe ou d'eucalyptus dans l'intervalle allant d'environ 0,3 à environ 0,75% en poids.

3. Agent de coloration selon la revendication 1 ou 2, **caractérisé en ce qu'**il contient du lactose dans l'intervalle allant d'environ 92 à 95% en poids, la phloxine B dans l'intervalle allant d'environ 2 à environ 2,2% en poids, le Bleu FDA N°1 dans l'intervalle allant d'environ 2,7 à environ 3,1% en poids, ainsi que le stéarate de magnésium dans l'intervalle allant d'environ 0,18 à environ 0,22% en poids.

4. Agent de coloration selon la revendication 3, **caractérisé en ce qu'**il contient du lactose dans l'intervalle d'environ 94,3% en poids, la phloxine B dans l'intervalle d'environ 2,1% en poids, le Bleu FDA N°1 dans l'intervalle d'environ 2,9% en poids, ainsi que le stéarate de magnésium dans l'intervalle d'environ 0,2% en poids.

5. Agent de coloration selon l'une des revendications 1 à 4, **caractérisé en ce qu'**il contient un arôme de menthe ou d'eucalyptus dans l'intervalle de 0,5% en poids.

6. Procédé de préparation d'un agent de coloration selon l'une des revendications 1 à 5, par compression directe, **caractérisé en ce que** l'on mélange l'un avec l'autre, des particules micronisées des colorants phloxine B et bleu FDA N°1 avec les auxiliaires de compression, le lactose et le stéarate de magnésium, ainsi que le cas échéant, un arôme de menthe ou d'eucalyptus et après la compression, on soumet les comprimés à une atmosphère saturée de vapeur d'eau, pendant environ 2 à 3 minutes.

7. Procédé selon la revendication 6, **caractérisé en ce que** l'on mélange l'un avec l'autre, du lactose dans l'intervalle allant d'environ 85 à 95% en poids, la phloxine B dans l'intervalle allant d'environ 1,5 à environ 2,6% en poids, le Bleu FDA N°1 dans l'intervalle allant d'environ 2,5 à environ 3,5% en poids, ainsi que le stéarate de magnésium dans l'intervalle allant d'environ 0,15 à environ 0,25% en poids, et le cas échéant, un arôme de menthe ou d'eucalyptus dans l'intervalle allant d'environ 0,3 à environ 0,7% en poids, et ce mélange est compressé.
